(19) Europäisches Patentamt
European Patent Office
Office européen des brevets

(11) EP 3 981 382 B1

(12) **EUROPEAN PATENT SPECIFICATION**

(45) Date of publication and mention of the grant of the patent:
**04.03.2026 Bulletin 2026/10**

(21) Application number: **20818473.9**

(22) Date of filing: **04.06.2020**

(51) International Patent Classification (IPC):
*A61K 8/02* (2006.01) *A61K 8/26* (2006.01)
*A61K 8/58* (2006.01) *A61K 33/00* (2006.01)
*A61K 33/08* (2006.01) *A61K 33/30* (2006.01)
*A61P 17/16* (2006.01) *A61K 8/19* (2006.01)
*A61K 8/25* (2006.01) *A61K 8/27* (2006.01)
*A61K 8/29* (2006.01) *A61K 8/81* (2006.01)
*A61Q 17/00* (2006.01) *B82Y 5/00* (2011.01)

(52) Cooperative Patent Classification (CPC):
(C-Sets available)
**A61P 17/16; A61K 8/0241; A61K 8/25; A61K 8/26; A61K 8/27; A61K 8/29; A61K 8/585; A61K 8/8147; A61K 8/8158; A61K 8/8176; A61K 33/00; A61K 33/08; A61K 33/30; A61Q 17/00;**
A61K 2800/412; (Cont.)

(86) International application number:
**PCT/JP2020/022178**

(87) International publication number:
**WO 2020/246557 (10.12.2020 Gazette 2020/50)**

(54) **METHOD FOR PREVENTING ADHESION OF ATMOSPHERIC TOXIC SUBSTANCE**

VERFAHREN ZUR VERHINDERUNG DER ADHÄSION VON ATMOSPHÄRISCHEN TOXISCHEN SUBSTANZEN

PROCÉDÉ POUR EMPÊCHER L’ADHÉRENCE D’UNE SUBSTANCE TOXIQUE ATMOSPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.06.2019 JP 2019106341**

(43) Date of publication of application:
**13.04.2022 Bulletin 2022/15**

(73) Proprietor: **Kao Corporation**
**Chuo-ku**
**Tokyo 103-8210 (JP)**

(72) Inventors:
- **FUJII, Tomoya**
  **Tokyo 131-8501 (JP)**
- **TAKIZAWA, Hiroyuki**
  **Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**WO-A1-2018/182255** **JP-A- 2005 336 056**
**JP-A- 2006 008 980** **JP-A- 2006 510 679**
**JP-A- 2006 510 679** **JP-A- 2010 047 522**
**JP-A- H06 227 961** **JP-A- H06 227 961**
**US-A1- 2002 161 104** **US-A1- 2002 192 178**
**US-A1- 2004 126 341** **US-A1- 2004 126 341**

- **DATABASE WPI Week 200602, Derwent World Patents Index; AN 2006-014363, XP002809407**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 981 382 B1

(52) Cooperative Patent Classification (CPC): (Cont.)
A61K 2800/621; A61K 2800/624

C-Sets
**A61K 33/00, A61K 2300/00;**
**A61K 33/08, A61K 2300/00;**
**A61K 33/30, A61K 2300/00**

## Description

FIELD OF THE INVENTION

The present invention relates to a method for suppressing adhesion of air harmful substances.

BACKGROUND OF THE INVENTION

Recently, harmful substances floating in air such as pollens of cedar and Japanese cypress, air pollutants such as smoke and dust, and yellow sand (hereinafter also referred to as "air harmful substances") have become problematic because they cause various health harm to the human body. Among air harmful substances, particulate matters having a diameter of 2.5 μm or less, called PM 2.5, are composed of a carbon material, a sulfate, a nitrate, an ammonium salt, etc., as constituent components, and it is known that PM 2.5 and yellow sand cause diseases of circulatory system and respiratory systems by inhalation. In addition, it is pointed out that PM 2.5 as well as pollens and yellow sand adhere to or penetrate through skin to cause skin troubles. For example, Nature Chemistry, 3, 291-295 (2011) by Shiraiwa, et al (NPL 1). discloses an academic report relating to PM 2.5 that causes damage to skin. Consequently, demands for cosmetic materials capable of protecting skin from air harmful substances have increased.

For example, WO2014/136993 (PTL 1) describes a skin-care cosmetic material containing a specific amount of magnesium metasilicate aluminate and a specific amount of a UV protectant, as a skin-care cosmetic material capable of protecting skin from external stimulations such as air pollutants.

JP 2005 336056 A describes a pollen adsorption-preventing agent that comprises a powder having a phosphorylcholine group represented by formula (1) covalently bonded directly to the surface thereof.

(1)

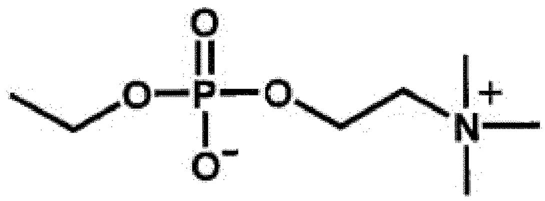

SUMMARY OF THE INVENTION

The present invention relates to a method for suppressing adhesion of air harmful substances, including applying an external preparation to skin to suppress adhesion of air harmful substances to the skin, wherein:
the external preparation contains the following component (A) and component (B), the content of the component (A) in the external preparation is 2 to 10% by mass, and the ratio by mass of the content of the component (A) to the content of the component (B) in the external preparation [(A)/(B)] is 0.30 or more and 5.0 or less:

Component (A): a metal oxide having an average primary particle diameter $d_A$ of 10 to 50 nm,
Component (B): non-disintegrable particles having an average particle diameter $D_B$ of 1 μm or more and 10 μm or less,
the component (A) is surface-hydrophobized,
the component (B) is at least one selected from the group consisting of

composite particles (B1) formed by coating at least a part of the surfaces of core particles (b1-1) that are at least one selected from the group consisting of poly(meth)acrylate particles and silica particles with inorganic fine particles (b1-2) that are at least one selected from the group consisting of titanium oxide and zinc oxide surface-treated with at least one selected from the group consisting of silica, hydrous silica and aluminum hydroxide, and porous particles (B2) formed by aggregation of inorganic fine particles (b2) having an average primary particle diameter of 1 nm or more and 30 nm or less.

BRIEF DESCRIPTION OF THE DRAWING

Fig. 1 is an outline view showing an evaluation method for the effect of suppressing adhesion of air harmful substances.

DETAILED DESCRIPTION OF THE INVENTION

PTL 1 describes that, according to the technique therein, magnesium metasilicate aluminate adsorbs air pollutants by the adsorption performance thereof to prevent them from reaching skin and, in addition, even when acid substances adhere to skin, magnesium metasilicate aluminate can neutralize them by the pH buffering capability thereof, and accordingly skin damage due to these external stimulations can be thereby relieved. In addition, it also describes that a UV protectant can effectively protect skin from UV rays. According to the technique, influences of air pollutants on skin can be reduced but air pollutants cannot be prevented from adhering to skin, and there is room for improvement. In the case where an inorganic powder such as titanium oxide or zinc oxide is blended in an external preparation as a UV protective agent, the external preparation often gives a poor feel after application to skin, and is especially poor in a dry silky feel. Consequently, a good feel after application to skin is also desired.

The present invention relates to a method for suppressing adhesion of air harmful substances having a high adhesion suppressing effect against air harmful substances and excellent in a feel after application to skin, especially in a dry silky feel.

The present inventors have noted that, not by adsorbing air pollutants as shown in PTL 1, but by applying an external preparation that contains a metal oxide having an average primary particle diameter falling within a specific range, and non-disintegrable particles having an average particle diameter falling within a specific range with a specific mass ratio, to skin to form nano-size irregularities on the surface of the skin without worsening the feel in application thereof to thereby suppress adhesion of air harmful substances to skin, and have found out a possibility of providing a method for suppressing adhesion of air harmful substances having a high adhesion suppressing effect against air harmful substances and excellent in a feel after application to skin, especially in a dry silky feel.

Specifically, the present invention relates to the following aspects [1] and [2].

[1] A method for suppressing adhesion of air harmful substances, including applying an external preparation to skin to suppress adhesion of air harmful substances to the skin, wherein:
the external preparation contains the following component (A) and component (B), the content of the component (A) in the external preparation is 2 to 10% by mass, and the ratio by mass of the content of the component (A) to the content of the component (B) in the external preparation [(A)/(B)] is 0.30 or more and 5.0 or less:

Component (A): a metal oxide having an average primary particle diameter $d_A$ of 10 to 50 nm,
Component (B): non-disintegrable particles having an average particle diameter $D_B$ of 1 μm or more and 10 μm or less,
the component (A) is surface-hydrophobized,
the component (B) is at least one selected from the group consisting of

composite particles (B1) formed by coating at least a part of the surfaces of core particles (b1-1) that are at least one selected from the group consisting of poly(meth)acrylate particles and silica particles with inorganic fine particles (b1-2) that are at least one selected from the group consisting of titanium oxide and zinc oxide surface-treated with at least one selected from the group consisting of silica, hydrous silica and aluminum hydroxide, and
porous particles (B2) formed by aggregation of inorganic fine particles (b2) having an average primary particle diameter of 1 nm or more and 30 nm or less.

[2] An external preparation containing the following component (A) and component (B):

Component (A): a metal oxide having an average primary particle diameter $d_A$ of 10 to 50 nm,
Component (B): non-disintegrable particles having an average particle diameter $D_B$ of 1 μm or more and 10 μm or less, wherein:

the component (B) is composite particles (B1) formed by coating at least a part of the surface of core particles (b1-1) with inorganic fine particles (b1-2),
the core particles (b1-1) are at least one selected from the group consisting of poly(meth)acrylate particles and silica particles,
the surface of the core particles (b1-1) is coated with at least one kind of binder (c) selected from the group consisting of a poly(N-vinylpyrrolidone), a poly(meth)acrylamide, and a homopolymer or a copolymer of oxazolines,
the inorganic fine particles (b1-2) are those of at least one selected from the group consisting of titanium oxide and zinc oxide as surface-treated with at least one selected from the group consisting of silica, hydrous silica

and aluminum hydroxide,
the content of the component (A) in the external preparation is 2 to 10% by mass, and
the ratio by mass of the content of the component (A) to the content of the component (B), [(A)/(B)] is 0.30 or more and 5.0 or less.

According to the present invention, there can be provided a method for suppressing adhesion of air harmful substances having a high adhesion suppressing effect against air harmful substances and excellent in a feel after application to skin, especially in a dry silky feel.

[Adhesion Suppressing Method against Air Harmful Substances]

The adhesion suppressing method against air harmful substances of the present invention is a method for suppressing adhesion of air harmful substances, including applying an external preparation to skin to suppress adhesion of air harmful substances to the skin, wherein:
the external preparation contains the following component (A) and component (B), the content of the component (A) in the external preparation is 2 to 10% by mass, and the ratio by mass of the content of the component (A) to the content of the component (B) in the external preparation [(A)/(B)] is 0.30 or more and 5.0 or less:

Component (A): a metal oxide having an average primary particle diameter $d_A$ of 10 to 50 nm,
Component (B): non-disintegrable particles having an average particle diameter $D_B$ of 1 μm or more and 10 μm or less,
the component (A) is surface-hydrophobized,
the component (B) is at least one selected from the group consisting of

composite particles (B1) formed by coating at least a part of the surfaces of core particles (b1-1) that are at least one selected from the group consisting of poly(meth)acrylate particles and silica particles with inorganic fine particles (b1-2) that are at least one selected from the group consisting of titanium oxide and zinc oxide surface-treated with at least one selected from the group consisting of silica, hydrous silica and aluminum hydroxide, and porous particles (B2) formed by aggregation of inorganic fine particles (b2) having an average primary particle diameter of 1 nm or more and 30 nm or less.

In the present invention, "air harmful substances" mean harmful substances (including PM 2.5) floating in air, such as pollens of cedar and Japanese cypress; air pollutants such as particles containing soot and smoke containing such as sulfur oxide, soot and dust, and nitrogen oxides, powder dust, motor exhaust, hazardous air pollutants such as benzene, trichloroethylene and tetrachloroethylene, and volatile organic compounds (VOC), etc.; and yellow sand.
The adhesion suppressing effect of the present invention provides a high adhesion suppressing effect against air harmful substances, especially against fine particulate air harmful substances, and is excellent in a feel after application to skin (especially in a dry silky feel). Though not clear, the reason may be considered as follows.
In the present invention, a metal oxide having an average primary particle diameter falling within a specific range is applied to skin to form nano-size irregularities on the surface of the skin, and accordingly, the contact area between air harmful substances, and the metal oxide in contact thereof can be reduced and adhesion of the air harmful substances can be thereby effectively suppressed.
The external preparation for use in the present invention contains the above-mentioned metal oxide and non-disintegrable particles having an average particle size falling within a predetermined range in a specific mass ratio. When applied onto the surface of skin, the non-disintegrable particles are hardly disintegrated even though rubbed and can maintain a particle size of a predetermined size, and consequently, it is considered that the external preparation can express a good feel (dry silky feel).
In the following description, the adhesion suppressing effect against air harmful substances in the present invention may be simply expressed as "adhesion suppressing effect", and the feel (dry silky feel) in the present invention may be simply a "feel".

<External Preparation>

[Component (A)]

The component (A) is a metal oxide having an average primary particle diameter $d_A$ of 10 to 50 nm.
The average primary particle diameter $d_A$ of the component (A) is, from the viewpoint of improving the adhesion suppressing effect, 50 nm or less, and is, from the viewpoint of general versatility, 10 nm or more. More specifically, the

average primary particle diameter $d_A$ is, from the viewpoint of improving the adhesion suppressing effect and from the viewpoint of general versatility, 10 to 50 nm.

The average primary particle diameter $d_A$ in the present invention can be determined on the observation image with a transmission electron microscope (TEM). Specifically, the particles are observed with TEM under the condition of an observation magnification power of 50,000 times, the maximum minor diameters of 300 primary particles on the observation image are measured, and the data are averaged to give a number-average value. Here, the maximum minor diameter means a minor diameter having a maximum length of minor diameters perpendicular to major diameter in the case where the component (A) has a shape different from a tabular one. In the case where the component (A) is a tabular one, the thicknesses of 300 primary particles in the image observed under the same condition as above are measured, and the data are averaged to give a number-average value. Specifically, the average primary particle diameter is measured according to the method described in the section of Examples.

The component (A) is not particularly limited as long as it can be generally used in external preparations such as cosmetic materials. Specific examples of the metal oxide (A) include titanium oxide, zinc oxide, cerium oxide, aluminum oxide (alumina), magnesium oxide, calcium oxide, zirconium oxide, iron oxide, and chromium oxide. Among these, from the viewpoint of giving UV protecting performance, preferred are one or more selected from the group consisting of titanium oxide, zinc oxide and cerium oxide, and more preferred are at least one selected from the group consisting of titanium oxide and zinc oxide, and from the viewpoint of further improving the adhesion suppressing effect, even more preferred is titanium oxide.

In the case where the component (A) is titanium oxide, the crystal structure of titanium oxide may be any of an anatase-type, rutile-type or Brookite-type one, but is, from the viewpoint of general versatility, preferably a rutile-type or anatase-type one.

The shape of the component (A) includes spherical, spindle-shaped, tabular and vesicular ones. Among these, from the viewpoint of improving the adhesion suppressing effect, a spherical, spindle-shaped or tabular one is preferred.

In the case where the component (A) is titanium oxide, preferably it is a spindle-shaped one from the viewpoint of improving the adhesion suppressing effect.

In the case where the component (A) is zinc oxide, preferably it is a spherical or tabular one from the viewpoint of improving the adhesion suppressing effect.

The existence form of the component (A) may be in a form of primary particles or may also be in a form containing aggregates formed by aggregating primary particles (secondary particles), so far as the average primary particle diameter $d_A$ thereof satisfies the above-mentioned range.

The component (A) is a surface-treated one from the viewpoint of enhancing the dispersibility of the component (A) in the external preparations to thereby improve the adhesion suppressing effect thereof. The surface treatment is a hydrophobizing treatment from the same viewpoint as above.

The hydrophobizing treatment includes a silicone treatment; an alkylalkoxysilane treatment; a fatty acid treatment; a fluorine-containing compound treatment with a perfluoroalkyl phosphate, a perfluoroalcohol or a perfluoroalkylalkoxysilane; an amino acid treatment with an N-acylglutamic acid; and an alkyl phosphate treatment.

One or more of these surface treatments may be carried out either singly or as combined.

Among these, from the viewpoint of enhancing the dispersibility of the component (A) in the external preparations to thereby improve the adhesion suppressing effect, at least one selected from the group consisting of a silicone treatment, an alkylalkoxysilane treatment and a fatty acid treatment are preferred.

The surface treatment agent for use in the silicone treatment includes various silicone oils such as methylpolysiloxane, dimethylpolysiloxane, methylphenylpolysiloxane, methylhydrogen polysiloxane, methylcyclopolysiloxane, dodecamethylcyclohexasiloxane, tetradecamethylhexasiloxane, dimethylsiloxane/methyl(polyoxyethylene)siloxane/methyl(polyoxypropylene)silox ane copolymer, dimethylsiloxane/methyl(polyoxyethylene)siloxane copolymer, dimethylsiloxane/methyl(polyoxypropylene)siloxane copolymer, dimethylsiloxane/methylcetyloxysiloxane copolymer, dimethylsiloxane/methylstearoxysiloxane copolymer, and (alkyl acrylate/dimethicone) copolymer. Among these, from the viewpoint of enhancing the dispersibility of the component (A) in the external preparation to improve the adhesion suppressing effect, preferred are methylhydrogen polysiloxane and dimethylpolysiloxane.

The surface treatment agent for use in the alkylalkoxysilane treatment is, from the viewpoint of enhancing the dispersibility of the component (A) in the external preparation to improve the adhesion suppressing effect, preferably one having a linear or branched alkyl group having 6 or more and 20 or less carbon atoms, more preferably octyltriethoxysilane or octyltrimethoxysilane.

The surface treatment agent for use in the fatty acid treatment includes a linear or branched fatty acid having 12 or more and 22 or less carbon atoms. Above all, from the viewpoint of enhancing the dispersibility of the component (A) in the external preparation to improve the adhesion suppressing effect, preferred is a linear or branched higher fatty acid having 14 or more and 22 or less carbon atoms, even more preferred is a linear or branched high fatty acid having 16 or more and 20 or less carbon atoms, and even more preferred are stearic acid and isostearic acid.

One or more of the above-mentioned surface treatment agents may be carried out either singly or as combined.

The amount of the hydrophobization treatment for the component (A) is, from the viewpoint of enhancing the dispersibility of the component (A) in the external preparation to improve the adhesion suppressing effect, preferably 0.1% by mass or more, and is preferably 40% by mass or less, more preferably 30% by mass or less, on the basis of the metal oxide (A).

For the surface-hydrophobized component (A), the mass, the coating amount and the average primary particle diameter $d_A$ of the component (A) mean the mass, the coating amount and the average primary particle diameter $d_A$, respectively, of the component (A) inclusive of the surface treatment agent.

In the case where the component (A) is titanium oxide, the content of $TiO_2$ in titanium oxide is, from the viewpoint of improving the adhesion suppressing effect, preferably 60% by mass or more, more preferably 70% by mass or more, and is preferably 100% by mass or less.

In the case where the component (A) is zinc oxide, the content of ZnO in zinc oxide is, from the viewpoint of improving the adhesion suppressing effect, preferably 60% by mass or more, more preferably 70% by mass or more, and is preferably 100% by mass or less.

The component (A) is, from the viewpoint of enhancing the dispersibility of the component (A) in the external preparation to improve the adhesion suppressing effect, preferably at least one selected from the group consisting of a hydrophobized titanium oxide and a hydrophobized zinc oxide, more preferably at least one selected from the group consisting of a silicone-treated titanium oxide, a silicone-treated zinc oxide, an alkylalkoxysilane-treated titanium oxide, an alkylalkoxysilane-treated zinc oxide, a fatty acid-treated titanium oxide and a fatty acid-treated zinc oxide, even more preferably at least one selected from the group consisting of a fatty acid-treated titanium oxide and an alkylalkoxysilane-treated zinc oxide.

Commercial products of the component (A) include "JR-800S" (silicone-treated titanium oxide), "MPY-70M" (silicone-treated titanium oxide), "MZ-504R3M" (silicone-treated zinc oxide), "MT-600KS" (silicone-treated zinc oxide), "MT-100TV" (stearic acid-treated titanium oxide), and "MT-100Z" (stearic acid-treated titanium oxide) all by Tayca Corporation; "MPT-171" (stearic acid-treated titanium oxide) by Ishihara Sangyo Kaisha Ltd.; "D-FZN" (silicone-treated zinc oxide) by Daito Kasei Corporation; "STR-100A-LP" (silicone-treated titanium oxide), "FINEX-50-LPTM" (silicone-treated zinc oxide), "FINEX-30-OTS" (octyltriethoxysilane-treated zinc oxide), "STR-100C-LF" (stearic acid-treated titanium oxide),"STR-100W-OTS" (octyltriethoxysilane-treated titanium oxide" and "FINEX-50-OTS" (octyltriethoxysilane-treated zinc oxide) all by Sakai Chemical Industry Co., Ltd.

[Component (B)]

The component (B) is non-disintegrable particles having an average particle diameter $D_B$ of 1 μm or more and 10 μm or less.

In the present invention, "non-disintegrable particles" are particles hardly disintegrable when rubbed under a predetermined load given thereto, and are specifically particles which are such that, when rubbed for ten rounds of reciprocation using a surface property tester Tribogear Type 14 (by Shinto Scientific Co., Ltd.), the particle diameter ratio before and after rubbing represented by the following formula (I) is 50% or more in a test method for confirming disintegrability described in the section of Examples.

$$\text{Particle Diameter Ratio before and after rubbing (\%)} = (\text{average particle diameter } D_B' \text{ after rubbing/ average particle diameter } D_B \text{ before rubbing}) \times 100 \quad (1)$$

The average particle diameter $D_B$ is, from the viewpoint of improving the feel after application to skin, 1 μm or more, preferably 2 μm or more, more preferably 3 μm or more, and is, from the viewpoint of improving the adhesion suppressing effect, 10 μm or less, preferably 9 μm or less, more preferably 8 μm or less. A specific range of the average particle diameter $D_B$ is, from the viewpoint of enhancing the adhesion suppressing effect and from the viewpoint of bettering the feel after application to skin, preferably 1 to 9 μm, more preferably 2 to 9 μm, even more preferably 3 to 9 μm, further more preferably 3 to 8 μm. The average particle diameter $D_B$ is measured according to the method described in the section of Examples.

The component (B) is preferably at least one selected from the group consisting of inorganic particles and organic particles.

The inorganic particles include particles containing at least one kind of inorganic substance selected from the group consisting of a silicon-containing compound such as silicon dioxide (silica), silicon nitride, silicon carbide, aluminum silicate, magnesium silicate, and calcium silicate; a metal oxide such as titanium oxide, zinc oxide, aluminum oxide (alumina), magnesium oxide, calcium oxide, zirconium oxide, iron oxide, cerium oxide, and chromium oxide; a metal salt such as magnesium carbonate, calcium carbonate, barium sulfate, barium carbonate, magnesium hydrogencarbonate, calcium hydrogencarbonate, lithium carbonate, calcium phosphate, titanium phosphate, silver chloride, and silver

bromide; a metal hydroxide such as magnesium hydroxide, aluminum hydroxide, and calcium hydroxide; a metal such as titanium, iron, chromium, nickel, gold, silver, platinum, copper, lead, and zinc, and a metal alloy thereof; diamond; a boron-containing compound such as boron nitride, and boron carbide; a metal carbide such as titanium carbide, tantalum carbide, and zirconium carbide; a metal nitride such as aluminum nitride, and titanium nitride; and a mixture of magnesium metasilicate aluminate (magnesium silicate aluminate). One kind alone or two or more kinds of these inorganic particles can be used either singly or as combined.

The inorganic particles may not be surface-treated or may be surface-treated.

The content of the inorganic substance in the inorganic particles is preferably 60% by mass or more, more preferably 70% by mass or more, and is preferably 100% by mass or less.

In the case where the inorganic particles are surface-treated ones, the mass and the average particle diameter $D_B$ of the inorganic particles mean the mass and the average particle diameter $D_B$ thereof inclusive of the surface treatment agent.

In the case where the component (B) is inorganic particles, the component (B) is, among the above, from the viewpoint of improving the feel after application to skin, preferably particles containing at least one selected from the group consisting of silicon-containing compound, a metal oxide, a metal salt, a metal nitride and a metal carbonate, more preferably particles containing one or more selected from a silicon-containing compound and a metal oxide, even more preferably particles containing at least one selected from the group consisting of silica, calcium silicate, titanium oxide, zinc oxide, and magnesium metasilicate aluminate, even more preferably particles containing at least one selected from the group consisting of silica and calcium silicate, further more preferably silica-containing silica particles.

The organic particles are, from the viewpoint of improving the feel after application to skin, polymer particles of poly(meth)acrylate. The polymer particles may be polymer particles having a crosslinked structure, or may also be polymer particles not having a crosslinked structure.

In this description, "(meth)acrylate" means one or more selected from acrylate and methacrylate.

The poly(meth)acrylate includes a homopolymer and a copolymer of a (meth)acrylic monomer. The (meth)acrylic monomer includes a (meth)acrylic acid; and a (meth)acrylate such as a (meth)acrylate having a hydrocarbon group derived from an aliphatic alcohol having 1 or more and 18 or less carbon atoms. One kind alone or two or more kinds of (meth)acrylic monomers can be used either singly or as combined.

In this description, "(meth)acrylic acid" means one or more selected from acrylic acid and methacrylic acid.

The poly(meth)acrylate may also be a crosslinked structure-having poly(meth)acrylate that has been further copolymerized with a polyfunctional (meth)acrylate such as ethylene glycol di(meth)acrylate, propylene glycol di(meth) acrylate and hexanediol di(meth)acrylate, in addition to (meth)acrylic acid and (meth)acrylate.

Specific examples of the poly(meth)acrylate include a polymethyl methacrylate and an acrylic acid/methyl acrylate copolymer, and the crosslinked structure-having (poly)acrylate includes a butyl acrylate/ethylene glycol dimethacrylate/sodium methacrylate copolymer, and a lauryl methacrylate/ethylene glycol dimethacrylate/sodium methacrylate copolymer.

The poly(meth)acrylate particles include particles that are described as "crosslinked (meth)acrylate-based resin particles" in JP 2006-8980 A, Gantz Pearl Series by Aica Kogyo Company, Limited.

The existence form of the component (B) may be a form of primary particles, or may also be a form containing aggregates of primary particles (secondary particles).

The particle structure of the component (B) includes porous particles and composite particles composed of core particles and fine particles to coat the surfaces of the core particles. In the present invention, "non-porous particles" mean particles having a specific surface area of 50 $m^2/g$ or less; and "porous particles" mean particles having a specific surface area of more than 50 $m^2/g$. The specific surface area can be measured according to JIS Z 8830:2013.

The component (B) is, from the viewpoint of improving the adhesion suppressing effect and from the viewpoint of improving the feel after application to skin, at least one selected from the group consisting of composite particles (B1) formed by coating at least a part of the surfaces of core particles (b1-1) with inorganic fine particles (b1-2), and porous particles (B2).

(Composite Particles (B1))

The composite particles (B1) are particles formed by coating at least a part of the surfaces of core particles (b1-1) with inorganic fine particles (b1-2).

The core particles (b1-1) may be any of non-porous particles or porous particles.

The average particle diameter of the core particles (b1-1) is preferably 1 μm or more, more preferably 2 μm or more, even more preferably 3 μm or more, and is preferably 10 μm or less, more preferably 9 μm or less, even more preferably 8 μm or less. The average particle diameter of the core particles (b1-1) can be measured according to the method described in the section of Examples.

For the core particles (b1-1), any of the above-mentioned organic particles and inorganic particles can be used.

Among these, from the viewpoint of improving the adhesion suppressing effect and from the viewpoint of improving the feel after application to skin, at least one selected from the group consisting of poly(meth)acrylate particles and silica particles are preferred.

Commercial products of the core particles (b1-1) include poly(meth)acrylate particles such as particles described as "crosslinked (meth)acrylate-based resin particles" in JP 2006-8980 A, Gantz Pearl Series by Aica Kogyo Company, Limited.; and silica particles such as "NP-100" by AGC SI-Tec Co., Ltd.

The core particles (b1-1) are, from the viewpoint of productivity of composite particles, preferably those coated with a binder (c) on the surfaces thereof. With that, the composite particles (B1) can have a structure such that the inorganic fine particles (b1-2) have adsorbed to the core particles (b1-1) via the binder (c). The binder (c) is preferably one having a functional group capable of inducing an interaction with the surface hydroxy group of the inorganic fine particles (b1-2), more preferably a polymer having an amide bond or an amide group in the side chain. Using a polymer having an amide bond or an amide group in the side chain as the binder (c), a structure can be formed in which the inorganic fine particles (b1-2) have adsorbed to the surfaces of the core particles (b1-1) via a hydrogen bond.

The binder (c) is, from the same viewpoint as above, preferably at least one selected from the group consisting of a vinyl polymer obtained by addition polymerization of an amide bond or amide group-having vinyl monomer, and a homopolymer or a copolymer of oxazolines, more preferably at least one selected from the group consisting of a poly(N-vinylpyrrolidone), a poly(meth)acrylamide, and a homopolymer or a copolymer of oxazolines, even more preferably at least one selected from the group consisting of a poly(N-vinylpyrrolidone), and a homopolymer or a copolymer of oxazolines,

In this description, "(meth)acrylamide" means one or more selected from acrylamide and methacrylamide.

The vinyl monomer to constitute the vinyl polymer includes N-vinyl compounds such as N-vinylpyrrolidone, N-vinylacetamide, N-vinylformamide and N-vinylcaprolactam; and (meth)acrylamide-based monomers such as (meth) acrylamide, and N-alkyl(meth)acrylamide. Among these, preferred are at least one selected from the group consisting of poly(N-vinylpyrrolidone) and poly(meth)acrylamide; and more preferred is poly(N-vinylpyrrolidone).

The poly(N-vinylpyrrolidone) includes an N-vinylpyrrolidone homopolymer; and a copolymer of N-vinylpyrrolidone and any other monomer such as an N-vinylpyrrolidone/vinyl acetate copolymer and an N-vinylpyrrolidone/vinyl acetate/vinyl propionate copolymer. Commercial products of poly(N-vinylpyrrolidone) include PVP K-90 (by Ashland Specialty Ingredients Corporation); PVP K-25, PVP K-30, and PVP K-90 (all trade names by FUJIFILM Wako Chemicals Corporation); PVA-6450, and Akone M (both trade names by Osaka Organic Chemical Industry Ltd.); and Corydon VA64 (vinylpyrrolidone/vinyl acetate copolymer, trade name by BASF Japan Corporation).

The poly(meth)acrylamide includes a homopolymer of a (meth)acrylic monomer; and a copolymer of a (meth) acrylic monomer and any other monomer such as (meth)acrylate or (meth)acrylic acid, and a salt thereof. Examples of the poly(meth)acrylamide include an N-tert-butylacrylamide/N,N-dimethylacrylamide/N-[3-(dimethylamino)propyl]acrylamide/methoxypolyethylene glycol methacrylate copolymer described in JP 2005-162700 A.

The homopolymer or copolymer of oxazolines is, from the viewpoint of improving the feel after application to skin, preferably a copolymer of oxazolines, more preferably an oxazoline-modified silicone having a poly(N-acylalkyleneimine) chain in the side chain prepared by ring-cleavage polymerization of a cyclic iminoether.

The oxazoline-modified silicone includes a poly(N-acylalkyleneimine)/organopolysiloxane copolymer such as a poly(N-formylethyleneimine)organopolysiloxane copolymer, a poly(N-acetylethyleneimine)organopolysiloxane copolymer, and a poly(N-propionylethyleneimine)/organopolysiloxane copolymer. Above all, preferred is a poly(N-propionylethyleneimine)/methylpolysiloxane copolymer (POLYSILICONE-9).

The poly(N-acylalkyleneimine)/organopolysiloxane copolymer can be obtained, for example, according to a method of reacting a poly(N-acylalkyleneimine) that is a ring cleavage polymer of a cyclic iminoether, and an organopolysiloxane to form a main chain segment. As POLYSILICONE-9, for example, those described in JP 2016-6029 A can be used.

The average primary particle diameter of the inorganic fine particles (b1-2) is preferably 1 nm or more, more preferably 2 nm or more, even more preferably 3 nm or more, and is preferably 30 nm or less, more preferably 25 nm or less, even more preferably 20 mm or less. The average primary particle diameter of the inorganic fine particles (b1-2) can be measured according to the method described in the section of Examples.

The inorganic fine particles (b1-2) include the above-mentioned inorganic particles.

The metal oxide used for the inorganic fine particles (b1-2) is a surface-hydrophilized one.

The hydrophilization treatment includes at least one selected from the group consisting of a silica treatment, a hydrous silica treatment and an aluminum hydroxide treatment.

The amount of hydrophilization treatment for the inorganic fine particles (b1-2) is preferably 0.1% by mass or more, and is preferably 40% by mass or less, more preferably 30% by mass or less, on the basis of the inorganic fine particles (b1-2).

The inorganic fine particles (b1-2) are, from the viewpoint of improving the feel after application to skin while improving the adhesion suppressing effect, at least one selected from the group consisting titanium oxide and zinc oxide

surface-treated with at least one selected from the group consisting of silica, hydrous silica and aluminum hydroxide.

Commercial products of the inorganic fine particles (b1-2) include "MT-100WP" (hydrous silica-treated titanium oxide fine particles) by Tayca Corporation; "STR-100W" (hydrous silica-treated titanium oxide fine particles", "STR-100C" (aluminum hydroxide-treated titanium oxide fine particles") and "FINEX-33W" (hydrous silica-treated zinc oxide fine particles) all by Sakai Chemical Industry Co., Ltd..

Preferably, the composite particles (B1) are produced by mixing the core particles (b1-1) and the inorganic fine particles (b1-2). In that manner, by heterogeneous aggregation of the core particles (b1-1) having a large average particle diameter and the inorganic fine particles (b1-2) having a small average primary particle diameter, a structure where at least a part of the surfaces of the core particles (b1-1) are coated with the inorganic fine particles (b1-2) can be formed.

The mixing method is not particularly limited as long as it can be generally used a method of adding the inorganic fine particles (b1-2) to a dispersion of the core particles (b1-1). The dispersant for the core particles (b1-1) is not specifically limited, and examples thereof include a lower alcohol such as ethanol; and water.

In the case where particles whose surfaces have been coated with a binder (c) are used as the core particles (b1-1), preferably, particles that are formed by previously mixing the particles to constitute the core particles (b1-1) and the binder (c) are used.

The blending amount of the binder (c) relative to the core particles (b1-1) is, from the viewpoint of productivity of the composite particles, preferably 0.01% by mass or more, more preferably 0.03% by mass or more, even more preferably 0.05% by mass or more, and is preferably 3% by mass or less, more preferably 1% by mass or less, even more preferably 0.5% by mass or less.

The blending amount of the inorganic fine particles (b1-2) relative to the core particles (b1-1) is, from the viewpoint of productivity of the composite particles, preferably 0.05% by mass or more, more preferably 0.15% by mass or more, even more preferably 0.25% by mass or more, and is preferably 10% by mass or less, more preferably 3% by mass or less, even more preferably 1% by mass or less.

(Porous Particles (B2))

The porous particles (B2) are those formed by aggregation of inorganic fine particles (b2) having an average primary particle diameter of 1 nm or more and 30 nm or less. The inorganic fine particles (b2) may be the same as the above-mentioned inorganic particles. Among them, from the viewpoint of improving the feel after application and improving the feel in use of the external preparation, preferred are particles containing at least one selected from the group consisting of a silicon-containing compound and a metal oxide; and more preferred are particles containing at least one selected from the group consisting of silica and titanium oxide.

The average primary particle diameter of the porous particles (B2) can be measured according to the method described in the section of Examples.

Commercial products of the porous particles (B2) include "Sunsphere H-51" (porous silica particles) by AGC SI-Tec Co., Ltd.; and "HCS Reflle 50" (aggregates of silica and titanium oxide) by JGC Catalysts and Chemical Ltd.

The external preparation can appropriately contain, in addition to the component (A) and the component (B) therein, beauty components and pharmaceutical components used in accordance with the intended use of the external preparation, and also any other components generally used in external preparations such as skin cosmetic materials, unless the objects of the present invention are adversely affected by inclusion thereof. The components include, except the component (A) and the component (B), an oily agent, a solvent, an antioxidant, a UV absorbent, a surfactant, a thickener, an emulsifier, a neutralizing agent, a pH regulator, a bactericide, an anti-inflammatory agent, a preservative, a colorant, a chelating agent, a moisturizer, a pearly agent, ceramides, an antiperspirant, and fragrances.

[Production of External Preparation]

The external preparation for use in the present invention can be produced by appropriately employing known methods in accordance with the form of the external preparation. For example, there is mentioned a method of blending the component (A), the component (B), and optionally the above-mentioned other components by stirring and mixing them with a disperser or the like.

In the case where the external preparation is a water-in-oil (W/O) type or an oil-in-water (O/W) type to be mentioned hereinunder, also employable is a method including preparing an aqueous phase and an oily phase and mixing the two. The content and the mass ratio of each component are as mentioned below.

(Content of Component (A) in External Preparation)

The content of the component (A) in the external preparation is, from the viewpoint of improving the adhesion suppressing effect, 2% by mass or more, and is, from the viewpoint of improving the feel after application to skin and from

the viewpoint of economy, 10% by mass or less. More specifically, the content of the component (A) is, from the viewpoint of improving the adhesion suppressing effect, from the viewpoint of improving the feel after application to skin and from the viewpoint of improving the feel in use when using the external preparation, 2 to 10% by mass.

(Content of Component (B) in External Preparation)

The content of the component (B) in the external preparation is, from the viewpoint of improving the feel after application to skin, preferably 0.1% by mass or more, more preferably 0.3% by mass or more, even more preferably 0.5% by mass or more, and is, from the viewpoint of improving the adhesion suppressing effect, preferably 40% by mass or less, more preferably 20% by mass or less, even more preferably 10% by mass or less.

(Mass Ratio [(A)/(B)])

The ratio by mass of the content of the component (A) to the content of the component (B) in the external preparation [(A)/(B)] is, from the viewpoint of improving the adhesion suppressing effect, 0.30 or more, preferably 0.32 or more, more preferably 0.35 or more, even more preferably 0.40 or more, further more preferably 0.60 or more, further more preferably 0.80 or more, and is, from the viewpoint of improving the feel after application to skin, 5.0 or less, preferably 4.5 or less, more preferably 4.0 or less, even more preferably 3.5 or less, further more preferably 3.0 or less, further more preferably 2.5 or less. More specifically, the mass ratio [(A)/(B)] is, from the viewpoint of improving the adhesion suppressing effect and from the viewpoint of improving the feel after application to skin, preferably 0.32 to 4.5, more preferably 0.35 to 4.0, even more preferably 0.40 to 3.5, further more preferably 0.60 to 3.0, further more preferably 0.80 to 2.5.

In the case where the external preparation contains an oily agent, the content of the oily agent in the external preparation is, from the viewpoint of improving the adhesion suppressing effect and from the viewpoint of improving the feel after application to skin, preferably 1% by mass or more, more preferably 3% by mass or more, even more preferably 7% by mass or more, and is preferably 40% by mass or less, more preferably 35% by mass or less, even more preferably 30% by mass or less.

The form of the external preparation for use in the present invention incudes a water-based type having one aqueous phase as a dispersion medium, an oil-based type having one oily phase as a dispersion medium, an oil-in-water type (O/W type), and a water-in-oil type (W/O type), and these can be appropriately selected.

In the present invention, the external preparation can be applied to, for example, skin or hair. The external preparation is, from the viewpoint of the adhesion suppressing effect, preferably a cosmetic material, more preferably a skin cosmetic material.

The preparation form of the external preparation is not specifically limited, and may be in any preparation form of a liquid form, a foamy form, a paste form, a cream form, or a solid form.

[Application Method]

In the present invention, regarding the method of applying the external preparation to skin, any known method is employable in accordance with the use form and the intended object of the external application. Here, "apply to skin" includes not only directly applying the external preparation to the surface of skin by hand, but also adhering the external preparation to the surface of skin by spraying or the like. The external preparation that is a liquid form, a foamy form, a paste form, a cream form or a solid form can be generally directly applied as it is, or can be applied by spraying or the like.

(Coating Amount of Component (A))

The coating amount of the component (A) on the surface of skin is, from the viewpoint of improving the adhesion suppressing effect, preferably 0.01 $mg/cm^2$ or more, more preferably 0.02 $mg/cm^2$ or more, even more preferably 0.03 $mg/cm^2$ or more, further more preferably 0.04 $mg/cm^2$ or more, and is, from the viewpoint of improving the feel after application to skin and from the viewpoint of economy, preferably 0.8 $mg/cm^2$ or less, more preferably 0.7 $mg/cm^2$ or less. More specifically, the coating amount of the component (A) on the surface of skin is, from the viewpoint of improving the adhesion suppressing effect and from the viewpoint of improving the feel after application to skin and also from the viewpoint of economy, preferably 0.01 to 0.8 $mg/cm^2$, more preferably 0.02 to 0.8 $mg/cm^2$, even more preferably 0.03 to 0.8 $mg/cm^2$, further more preferably 0.04 to 0.8 $mg/cm^2$, further more preferably 0.04 to 0.7 $mg/cm^2$.

In the case where the external preparation is a W/O type, the coating amount of the component (A) on the surface of skin is, from the viewpoint of enhancing the adhesion suppressing effect, preferably 0.01 $mg/cm^2$ or more, more preferably 0.02 $mg/cm^2$ or more, even more preferably 0.03 $mg/cm^2$ or more, further more preferably 0.04 $mg/cm^2$ or more, and is, from the viewpoint of improving the feel in use in application to skin and from the viewpoint of economy, preferably

0.8 mg/$cm^2$ or less, more preferably 0.7 mg/$cm^2$ or less. More specifically, the coating amount of the component (A) on the surface of skin is, from the viewpoint of improving the adhesion suppressing effect and from the viewpoint of improving the feel after application to skin, and also from the viewpoint of economy, preferably 0.01 to 0.8 mg/$cm^2$, more preferably 0.02 to 0.8 mg/$cm^2$, even more preferably 0.03 to 0.8 mg/$cm^2$, further more preferably 0.04 to 0.8 mg/$cm^2$, further more preferably 0.04 to 0.7 mg/$cm^2$.

In the case where the external preparation is an O/W type, the coating amount of the component (A) on the surface of skin is, from the viewpoint of improving the adhesion suppressing effect, preferably 0.01 mg/$cm^2$ or more, more preferably 0.02 mg/$cm^2$ or more, even more preferably 0.03 mg/$cm^2$ or more, further more preferably 0.04 mg/$cm^2$ or more, and is, from the viewpoint of improving the feel after application to skin and from the viewpoint of economy, preferably 0.8 mg/$cm^2$ or less, more preferably 0.7 mg/$cm^2$ or less, even more preferably 0.5 mg/$cm^2$ or less, further more preferably 0.3 mg/$cm^2$ or less. More specifically, the coating amount of the component (A) on the surface of skin is, from the viewpoint of improving the adhesion suppressing effect and from the viewpoint of improving the feel after application to skin, and also from the viewpoint of economy, preferably 0.01 to 0.8 mg/$cm^2$, more preferably 0.02 to 0.8 mg/$cm^2$, even more preferably 0.03 to 0.8 mg/$cm^2$, further more preferably 0.04 to 0.7 mg/$cm^2$, further more preferably 0.04 to 0.5 mg/$cm^2$, further more preferably 0.04 to 0.3 mg/$cm^2$.

Examples

In the following Examples and Comparative Examples, "part" and "%" are "part by mass" and "% by mass", respectively, unless otherwise specified.

(1) Average primary particle diameter $d_A$ of component (A) and average primary particle diameter of inorganic fine particles (b1-2) and porous particles (B2)

In the case where the measurement sample has any other shape than a tabular one, a dispersion of the measurement sample prepared previously was put on a sample stage of a transmission electron microscope (TEM) (trade name "JEM1400Plus" by JEOL Corporation), air-dried thereon, and the maximum minor diameter of each of 300 primary particles on the image taken by TEM at an observation magnification of 50,000x was measured, and the resultant data were averaged to give a number-average value to be the average primary particle diameter of the sample. Here, the maximum minor diameter means a minor diameter having a maximum length of minor diameters perpendicular to the major diameter.

In the case where the measurement sample is a tabular one, the thickness of each of 300 primary particles was measured on the image taken according to the same method and under the same observation magnification condition, and the resultant data were averaged to give a number-average value to be the average primary particle diameter of the sample.

A dispersion of the measurement sample was prepared by ultrasonically dispersing 5 g of the measurement sample in 95 g of ethanol as a solvent added thereto.

(2) Average particle diameter $D_B$ of component (B), and average particle diameter of core particles (b1-1)

Measured using a laser diffraction/scattering particle size distribution measurement apparatus (trade name "LA-920", by Horiba, Ltd.), and using ion-exchanged water as a dispersion medium. In measuring organic particles, the relative refractive index was 1.10, and in measuring inorganic particles, the relative refractive index was 1.09. For the measurement, a flow cell was used. Regarding the measurement conditions, the circulation rate was 1.5 with no ultrasonic irradiation, the distribution form was standard, and the particle diameter was on a volume basis.

(3) Confirmation of disintegrability

0.04 g of a dispersion of particles (dispersion medium: ethanol, solid concentration: 5%) was uniformly applied and spread on an artificial leather (trade name, "Laforet S2923" by Okamoto Shinwa Co., Ltd.) (5 cm × 4 cm) taking 20 seconds (amount of adhered particles: 0.1 mg/$cm^2$), and dried at room temperature for 24 hours to give measurement samples. Using a surface profilometer (trade name "Tribogear Type: 14", by Shinto Scientific Co., Ltd.) (vertical load: 360 g, moving distance: 50 mm, moving speed: 600 mm/min), the particles-coated surface of the measurement sample was rubbed for 10 reciprocations with a tool (3 cm ×3 cm) having an uncoated artificial leather (Laforet S2923) (3 cm × 10 cm) attached thereto. Next, using a field emission scanning electron microscope (FE-SEM-4800 (5.0 kV)) (by Hitachi High-Tech Corporation), the rubbed surface of the measurement sample was observed at an observation magnification of 15,000x. The major diameter and the minor diameter of each of 30 particles on the observation image were measured, and an average value of the major diameter and the minor diameter was referred to as the particle diameter of each particles.

From the found data of the particle diameter of 30 particles, a volume-based particle diameter was calculated to be the average particle diameter $D_B$' after rubbing.

With reference to the average particle diameter $D_B$' after rubbing and the average particle diameter $D_B$ before rubbing as measured in the above (2), particles having a particle diameter ratio represented by the following formula (I) of less than 50% were referred to as "easily disintegrable particles" and particles having a particle diameter ratio represented by the following formula (I) of 50% or more were referred to as "non-disintegrable particles".

Particle Diameter Ratio before and after rubbing (%) = (average particle diameter $D_B$' after rubbing/ average particle diameter $D_B$ before rubbing) $\times$ 100 (I) (I)

(Production of polymethacrylate particles P1)

Production Example 1

According to Example 1 in JP 2006-8980 A, polymethacrylate particles P1 (particles of lauryl methacrylate/ethylene glycol dimethacrylate/sodium methacrylate copolymer) were synthesized. The polymethacrylate particles P1 had an average particle diameter of 3 $\mu$m.

(Preparation of composite particles (B1))

Preparation Example 1

100 g of core particles (b1-1), the polymethacrylate particles P1 obtained in Production Example 1 were dispersed in 150 g of pure water, then 0.1 g of an oxazoline-modified silicone (trade name "OS-50TE-E", by Kao Corporation) was added thereto, and stirred for 1 hour to give oxazoline-modified silicone-coated polymethacrylate particles P1-1 (hereinafter also expressed as "coated polymethacrylate particles P1-1"). Next, 0.5 g of inorganic fine particles (b1-2), fine particles of hydrous silica-treated titanium oxide shown in Table 1 were added, and stirred for 30 minutes to give composite particles B1-1. The composite particles B1-1 were used as the external preparation mentioned below, directly as they were unpurified.

Preparation Example 2

As core particles (b1-1), oxazoline-modified silicone-coated polymethacrylate particles P1-1 were prepared in the same manner as in Preparation Example 1, then 0.5 g of inorganic fine particles (b1-2), fine particles of aluminum hydroxide-treated titanium oxide shown in Table 1 were added, and stirred for 30 minutes to give composite particles B1-2. The composite particles B1-2 were used as the external preparation mentioned below, directly as they were unpurified.

Preparation Example 3

100 g of core particles (b1-1), the polymethacrylate particles P1 obtained in Production Example 1 were dispersed in 150 g of pure water, then 0.1 g of a polyvinylpyrrolidone (trade name "PVP K-90" by Ashland Specialty Ingredients Corporation) was added, and stirred for 1 hour to give polyvinylpyrrolidone-coated polymethacrylate particles P1-2 (hereinafter also expressed as "coated polymethacrylate particles P1-2"). Next, 0.5 g of inorganic fine particles (b1-2), fine particles of hydrophilic silica shown in Table 1 were added, and stirred for 30 minutes to give composite particles B1-3. The composite particles B1-3 were used as the external preparation mentioned below, directly as they were unpurified.

Preparation Example 4

100 g of core particles (b1-1), silica particles P2 (trade name "Sunsphere NP-100", by AGC SI-Tec Co., Ltd., average particle diameter 10 $\mu$m) were dispersed in 150 g of pure water, then 0.1 g of a polyvinylpyrrolidone (PVP K-90) was added, and stirred for 1 hour to give polyvinylpyrrolidone-coated silica particles P2-1 (hereinafter also expressed as "coated silica particles P2-1"). Next, 0.5 g of inorganic fine particles (b1-2), fine particles of hydrophilic silica shown in Table 1 were added, and stirred for 30 minutes to give composite particles B1-4. The composite particles B1-4 were used as the external preparation mentioned below, directly as they were unpurified.

Preparation Example 5

As core particles (b1-1), oxazoline-modified silicone-coated polymethacrylate particles P1-1 were prepared in the same manner as in Preparation Example 1, then 0.5 g of inorganic fine particles (b1-2), fine particles of hydrous silica-treated zinc oxide shown in Table 1 were added, and stirred for 30 minutes to give composite particles B1-5. The composite particles B1-5 were used as the external preparation mentioned below, directly as they were unpurified.

Preparation Example 6

100 g of core particles (b1-1), silica particles P3 (trade name "Sciqas", by Sakai Chemical Industry Co., Ltd., surface-untreated product, average particle diameter 0.4 μm) were dispersed in 150 g of pure water, then 0.1 g of a polyvinylpyrrolidone (PVP K-90) was added, and stirred for 1 hour to give polyvinylpyrrolidone-coated silica particles P3-1 (hereinafter also expressed as "coated silica particles P3-1"). Next, 0.5 g of inorganic fine particles (b1-2), fine particles of hydrophilic silica shown in Table 1 were added, and stirred for 30 minutes to give composite particles B1'-6. The composite particles B1'-6 were used as the external preparation mentioned below, directly as they were unpurified.

Preparation Example 7

As core particles (b1-1), polyvinylpyrrolidone-coated polymethacrylate particles P1-2 were prepared in the same manner as in Preparation Example 3, then 0.5 g of inorganic fine particles (b1-2), fine particles of hydrous silica-treated titanium oxide were added, and stirred for 30 minutes to give composite particles B1-7. The composite particles B1-7 were used as the external preparation mentioned below, directly as they were unpurified.

Table 1

| | | | Core Particles (b1-1) | | | Inorganic Fine Particles (b1-2) | |
|---|---|---|---|---|---|---|---|
| | | | Kind | Binder (c) *1 | Average Particle Diameter (μm) | Kind | Trade Name |
| Preparation Example | 1 | Composite Particles B1-1 | Coated Polymethacrylate Particles P1-1 | OxS | 3 | Fine Particles of hydrous silica-treated titanium oxide | Trade Name "STR-100W", by Sakai Chemical Industry Co., Ltd. |
| | 2 | Composite Particles B1-2 | Coated Polymethacrylate Particles P1-1 | OxS | 3 | Fine Particles of aluminum hydroxide-treated titanium oxide | Trade Name "STR-100C", by Sakai Chemical Industry Co., Ltd. |
| | 3 | Composite Particles B1-3 | Coated Polymethacrylate Particles P1-2 | PVP | 3 | Fine Particles of hydrophilic silica | Trade Name "AEROSIL 200", by Nippon Aerosil Co., Ltd. |
| | 4 | Composite Particles B1-4 | Coated Silica Particles P2-1 | PVP | 10 | Fine Particles of hydrophilic silica | Trade Name "AEROSIL 200", by Nippon Aerosil Co., Ltd. |
| | 5 | Composite Particles B1-5 | Coated Polymethacrylate Particles P1-1 | OxS | 3 | Fine Particles of hydrous silica-treated zinc oxide | Trade Name "FINEX-33W", by Sakai Chemical Industry Co., Ltd. |
| | 6 | Composite Particles B1'-6 | Coated Silica Particles P3-1 | PVP | 0.4 | Fine Particles of hydrophilic silica | Trade Name "AEROSIL 200", by Nippon Aerosil Co., Ltd. |
| | 7 | Composite Particles B1-7 | Coated Polymethacrylate Particles P1-2 | PVP | 3 | Fine Particles of hydrous silica-treated titanium oxide | Trade Name "STR-100W", by Sakai Chemical Industry Co., Ltd. |

*1: OxS is oxazoline-modified silicone (trade name "OS-50TE-E", by Kao Corporation; PVP is polyvinylpyrrolidone (trade name "PVP K-90", by Ashland Specialty Ingredients Corporation.)

Examples 1 to 12, Comparative Examples 1 to 7

At a temperature 25°C according to the formulation shown in Tables 2 to 4, the component (B) or the component (B'), and also a thickener, an emulsifier and a solvent were put into a water-containing formulation tank with stirring to prepare an aqueous phase. Separately, at a temperature 80°C, the component (A) and an oily agent were mixed to prepare an oily phase. Subsequently, the oily phase and a neutralizer were added to the aqueous phase and stirred to prepare external preparations according to the formulation of Tables 2 to 4.

The resultant external preparations were evaluated in point of the adhesion suppressing effect against air harmful substances and the feel, according to the methods shown below. The results are given in Tables 2 to 4.

Expressions in Tables 2 to 4 are as follows.

The average primary particle diameter $d_A$ of the component (A), and the average particle diameter $D_B$ of the component (B) or the component (B') and the disintegrability of particles are shown in Tables 2 to 4.

Fine particles of stearic acid-treated titanium oxide A1: trade name "MT-100TV", by Tayca Corporation, spindle-shaped fine particles of stearic acid-treated titanium oxide

Fine particles of octyltriethoxysilane-treated zinc oxide A2: "FINEX-30-OTS", by Sakai Chemical Industry Co., Ltd., spherical fine particles of octyltriethoxysilane-treated zinc oxide

Porous particles B2-1: trade name "HCS Reflle 50", by JGC Catalysts and Chemical Ltd., aggregates of 160-nm silica and 250-nm titanium oxide (silica/titanium oxide = 50/50), oil absorption amount 50 mL/100 g
Porous particles B2-2: trade name "Sunsphere H-51", by AGC SI-Tec Co., Ltd., specific surface area 800 $m^2/g$
Silica particles P'4: trade name "Sunsphere H-121", by AGC SI-Tec Co., Ltd.
Silica particles P'5: trade name "Sunsphere H-52", by AGC SI-Tec Co., Ltd.
Isopropyl palmitate: trade name "Exeparl IPP", by Kao Corporation
Acrylic acid/alkyl methacrylate copolymer 1: trade name "PEMULENTR-1", by Lubrizol Advanced Materials Corporation
Acrylic acid/alkyl methacrylate copolymer 2: trade name "PEMULENTR-2", by Lubrizol Advanced Materials Corporation

<Evaluation of adhesion suppressing effect against air harmful substances>

The external preparation prepared in the above was applied to a white artificial leather (trade name, "Laforet S2923" by Okamoto Shinwa Co., Ltd.) as a substitute for skin to have a coating amount of the component (A) shown in Tables 2 to 4, and left overnight at room temperature to be dried.

The surface of the artificial leather coated with the external preparation was exposed to an air flow environment of air harmful substances for evaluation, and using a colorimeter, the L*a*b* value was measured, and a color difference ΔE before and after exposure was measured according to the following method.

[Measurement of color difference ΔE]

Using a colorimeter (trade name "CM-2002" by Konica Minolta Corporation), the $L_1^*$, $a_1^*$and $b_1^*$ values of the surface of the artificial leather coated with the external preparation, before exposed to air harmful substances for evaluation were measured.

Separately, a fan (trade name "Silky Wind 9ZF002RH02", size: 129 × 106 × 83 mm, by Rhythm Co., Ltd.) and a wire sieve (test sieve by JIS Z 8801, frame dimension: ϕ100 × 45 H, opening: 106 μm, by Tokyo Screen Co., Ltd.) were fixed in a glove bag (part number "3-118-01", by AS ONE Corporation). The installation height of the wire sieve was 17 cm.

The artificial leather (5 cm × 4 cm) coated with the external preparation of the test sample was attached to a support so that the height of the lower end of the artificial leather was 11 cm. The distance between the coated surface of the artificial leather on the support and the fan was 15 cm, and as shown in Fig. 1, the coated surface of the artificial leather was set to be perpendicular to the blowing direction of the fan, and the height of the center of the blade of the fan was to be the same as the height of the center of the artificial leather.

The temperature in the glove bag was 25°C and the relative humidity therein was 57% RH. Using a brush for removing clogging from the wire sieve (trade name "JNB-5", brush diameter 53 μm, by Tokyo Screen Co., Ltd.), 50 mg of a graphite powder (trade name, "J-CPB", average particle diameter: 5.5 μm, by Nippon Graphite Industries, Ltd.), as air harmful substances for evaluation was, while classified, dropped down for 1 minute before the blowout port of the fan whose blowout grade was set at 1. In that manner, the surface of the artificial leather coated with the external preparation was exposed to the air flow environment of air harmful substances for evaluation.

Next, using the above colorimeter, the $L_2^*$, $a_2^*$, $b_2^*$ values of the exposed surface of the artificial leather were measured, and the color difference ΔE value was calculated according to the following formula (II-1).

$$\Delta E = [(L_1^*-L_2^*)^2+(a_1^*-a_2^*)^2+(b_1^*-b_2^*)^2]^{0.5} \quad \text{(II-1)}$$

The above operation was repeated three times for every sample, and the mean value of the color difference ΔE of the artificial leather coated with the external preparation of the test sample was referred to as ΔEt. Further, an artificial leather not coated with the external preparation as a standard sample was treated three times in the same manner as above, and the mean value of the color difference ΔE was referred to as ΔEs. According to the following formula (II-2), the adhesion suppressing ratio was calculated. Ahigher adhesion suppressing ratio indicates a more excellent adhesion suppressing effect against air harmful substances.

Adhesion suppressing ratio against air harmful substances(%) = 100 × (ΔEs -ΔEt)/ΔEs (II-2) (II-2)

<Feel (dry silky feel) Evaluation>

Three expert panelists tried external preparations by applying 0.02 mL of each external preparation to the inner portion of the forearm to be in a circular form having a diameter of 3 cm, followed by uniformly spreading it thereover under

the condition of 25°C and 57% RH, taking 20 seconds. In 15 minutes after application of the external preparation, the feel (dry silky feel) was organoleptically evaluated in 5 ranks according to the following criteria. An average score of the three panelists was calculated. The results are shown in Tables 2 to 4.

5: Much dry silky feel.
4: Dry silky feel.
3: No dry silky feel.
2: Sticky and squeaky feel.
1: Much sticky and squeaky feel.

Table 2

| | | Kind | Average Primary Particle Diameter $d_A$ (nm) | Average Particle Diameter $D_B$ (µm) | Disintegrability | Example | | | | | | | | |
|---|---|---|---|---|---|---|---|---|---|---|---|---|---|---|
| | | | | | | 1 | 2 | 3 | 4 | 5 | 6* | 7 | 8 | 9* |
| Formulation of External Preparation (part) | Component (A) | Fine Particles of stearic acid-treated titanium oxide A1 | 15 | - | - | 2 | 2 | 2 | 2 | 2 | 2 | 2 | 2 | 2 |
| | Component (B) | Polymethacrylate Particles P1 | - | 3 | non-disintegrable | 2 | 0.5 | 6 | | | | | | |
| | | Composite Particles B1-1 | - | 3 | non-disintegrable | | | | 2 | | | | | |
| | | Composite Particles B1-2 | - | 3 | non-disintegrable | | | | | 2 | | | | |
| | | Composite Particles B1-3 | - | 3 | non-disintegrable | | | | | | 2 | | | |
| | | Porous Particles B2-1 | - | 5 | non-disintegrable | | | | | | | 1 | | |
| | | Porous Particles B2-2 | - | 5 | non-disintegrable | | | | | | | | 1 | |
| | | Composite Particles B1-4 | - | 10 | non-disintegrable | | | | | | | | | 2 |
| | Oily Agent | Isopropyl Palmitate | | | | 10 | 10 | 10 | 10 | 10 | 10 | 10 | 10 | 10 |
| | Thickener/ Emulsifier | Acrylic Acid/Alkyl Methacrylate Copolymer 1 | | | | 0.25 | 0.25 | 0.25 | 0.25 | 0.25 | 0.25 | 0.25 | 0.25 | 0.25 |
| | | Acrylic Acid/Alkyl Methacrylate Copolymer 2 | | | | 0.1 | 0.1 | 0.1 | 0.1 | 0.1 | 0.1 | 0.1 | 0.1 | 0.1 |
| | | Polyoxyethylene 2-Hexyldecyl Ether | | | | 0.05 | 0.05 | 0.05 | 0.05 | 0.05 | 0.05 | 0.05 | 0.05 | 0.05 |
| | Neutralizer | Potassium Hydroxide | | | | 0.55 | 0.55 | 0.55 | 0.55 | 0.55 | 0.55 | 0.55 | 0.55 | 0.55 |
| | Solvent | Ethanol | | | | 5 | 5 | 5 | 5 | 5 | 5 | 5 | 5 | 5 |
| | | Pure Water | | | | 80.05 | 81.55 | 76.05 | 80.05 | 80.05 | 80.05 | 81.05 | 81.05 | 80.05 |
| | Total | | | | | 100 | 100 | 100 | 100 | 100 | 100 | 100 | 100 | 100 |
| Mass Ratio [(A)/(B)] | | | | | | 1.0 | 4.0 | 0.33 | 1.0 | 1.0 | 1.0 | 2.0 | 2.0 | 1.0 |
| Evaluation Results | Adhesion Suppressing Effect | Coating Amount of Component (A) (mg/cm²) | | | | 0.04 | 0.04 | 0.04 | 0.04 | 0.04 | 0.04 | 0.04 | 0.04 | 0.04 |
| | | Adhesion Suppressing Ratio (%) | | | | 54 | 60 | 48 | 62 | 65 | 61 | 67 | 71 | 73 |
| | Feel (dry silky feel) | | | | | 4.3 | 3.0 | 5.0 | 4.0 | 4.3 | 3.7 | 4.0 | 4.3 | 3.3 |

*Reference Examples

Table 3

| | | Kind | Average Primary Particle Diameter $d_A$ (nm) | Average Particle Diameter $D_B$ (µm) | Disintegrability | Comparative Example | | | | | | |
|---|---|---|---|---|---|---|---|---|---|---|---|---|
| | | | | | | 1 | 2 | 3 | 4 | 5 | 6 | 7 |
| Formulation of External Preparation | Component (A) | Fine Particles of stearic acid-treated titanium oxide A1 | 15 | - | - | 2 | | 2 | 2 | 2 | 2 | 4 |
| | Component (B) | Polymethacrylate Particles P1 | - | 3 | non-disintegrable | | 2 | | | | 8 | 0.75 |
| | Component (B') | Silica Particles P'4 | - | 12 | non-disintegrable | | | | 1 | | | |
| | | Composite Particles B1'-6 | - | 0.4 | non-disintegrable | | | 2 | | | | |
| | | Silica Particles P'5 | - | 5 | easily disintegrable | | | | | 1 | | |
| | Oily Agent | Isopropyl Palmitate | | | | 10 | 10 | 10 | 10 | 10 | 10 | 10 |
| | Thickener/ Emulsifier | Acrylic Acid/Alkyl Methacrylate Copolymer 1 | | | | 0.25 | 0.25 | 0.25 | 0.25 | 0.25 | 0.25 | 0.25 |
| | | Acrylic Acid/Alkyl Methacrylate Copolymer 2 | | | | 0.1 | 0.1 | 0.1 | 0.1 | 0.1 | 0.1 | 0.1 |
| | | Polyoxyethylene 2-Hexyldecyl Ether | | | | 0.05 | 0.05 | 0.05 | 0.05 | 0.05 | 0.05 | 0.05 |
| | Neutralizer | Potassium Hydroxide | | | | 0.55 | 0.55 | 0.55 | 0.55 | 0.55 | 0.55 | 0.55 |
| | Solvent | Ethanol | | | | 5 | 5 | 5 | 5 | 5 | 5 | 5 |
| | | Pure Water | | | | 82.05 | 82.05 | 80.05 | 81.05 | 81.05 | 74.05 | 79.3 |
| | Total | | | | | 100 | 100 | 100 | 100 | 100 | 100 | 100 |
| Mass Ratio [(A)/(B)] | | | | | | - | - | 1.0 | 2.0 | 2.0 | 0.25 | 5.3 |
| Evaluation Results | Adhesion Suppressing Effect | Coating Amount of Component (A) (mg/cm²) | | | | 0.04 | 0.04 | 0.04 | 0.04 | 0.04 | 0.04 | 0.04 |
| | | Adhesion Suppressing Ratio (%) | | | | 70 | -30 | 60 | 9 | 70 | 10 | 75 |
| | Feel (dry silky feel) | | | | | 1.7 | 5.0 | 2.0 | 4.7 | 1.7 | 5.0 | 2.0 |

Table 4

| | | Kind | Average Primary Particle Diameter $d_A$ (nm) | Average Particle Diameter $D_B$ (μm) | Disintegrability | Example 10 | Example 11 | Example 12 |
|---|---|---|---|---|---|---|---|---|
| Formulation of External Preparation (part) | Component (A) | Fine Particles of stearic acid-treated titanium oxide A1 | 15 | - | - | 2 | | |
| | | Fine Particles of octyltriethoxysilane-treated zinc oxide A2 | 35 | - | - | | 2 | 2 |
| | Component (B) | Porous Particles B2-2 | - | 5 | non-disintegrable | 1 | 1 | |
| | | Composite Particles B1-5 | - | 3 | non-disintegrable | | | 2 |
| | Oily Agent | Isopropyl Palmitate | | | | 10 | 10 | 10 |
| | | Isohexadecane | | | | 0.34 | 0.34 | 0.34 |
| | Thickener/ Emulsifier | Sodium Acrylate/Sodium Acryloyldimethyltaurate Copolymer | | | | 0.56 | 0.56 | 0.56 |
| | | Polysorbate 80 | | | | 0.11 | 0.11 | 0.11 |
| | | Sorbitan Oleate | | | | 0.38 | 0.38 | 0.38 |
| | Solvent | Ethanol | | | | 5 | 5 | 5 |
| | | Pure Water | | | | 80.61 | 80.61 | 79.61 |
| | Total | | | | | 100 | 100 | 100 |
| Mass Ratio [(A)/(B)] | | | | | | 2.0 | 2.0 | 1.0 |
| Evaluation Results | Adhesion Suppressing Effect | Coating Amount of Component (A) ($mg/cm^2$) | | | | 0.04 | 0.04 | 0.04 |
| | | Adhesion Suppressing Ratio (%) | | | | 66 | 58 | 56 |
| | Feel (dry silky feel) | | | | | 4.7 | 4.7 | 4.3 |

From Tables 2 to 4, it is known that, in Examples 1 to 12, the external preparation containing a metal oxide having an average primary particle diameter $d_A$ falling within a predetermined range as the component (A) and non-disintegrable particles having an average particle diameter $D_B$ falling within a predetermined range as the component (B) in a predetermined mass ratio was applied, and therefore, the samples had a higher adhesion suppressing effect and gave a better feel (dry silky feel) after application to skin, as compared with those in Comparative Example 1 to 7.

Examples 13, 14

At a temperature 25°C according to the formulation 1 or 2 shown in Table 5, the component (B) and a thickener, an emulsifier and a solvent were put into a water-containing formulation tank stirring to prepare an aqueous phase. Separately, at a temperature 80°C, the component (A) and an oily agent were mixed to prepare an oily phase. Subsequently, the oily phase and a neutralizer and other components were added to the aqueous phase and stirred to prepare external preparations according to the formulation of Table 5.

The resultant external preparations were evaluated in point of the adhesion suppressing effect against air harmful substances and the feel, according to the methods mentioned above. The results are given in Table 5.

In Table 5, expressions of the other components than those shown in Tables 2 to 4 are as follows. The average primary particle diameter $d_A$ of the component (A), and the average particle diameter $D_B$ and the disintegrability of the particle of the component (B) are shown in Table 5.

Fine particles of octyltriethoxysilane-treated titanium oxide A3: trade name "STR-100W-OTS", by Sakai Chemical Industry Co., Ltd., spindle-shaped fine particles of octyltriethoxysilane-treated titanium oxide

*1: trade name "Uvinul MC80", by BASF Japan Ltd.
*2: trade name "Uvinul T-150", by BASF Japan Ltd.
*3: trade name "TINOSORB S", by BASF Japan Ltd.
*4: trade name "Parleam EX" by NOF Corporation
*5: trade name "KF-96A-10CS" by Shin-Etsu Chemical Industry Co., Ltd.

*6: trade name "Cetyl Alcohol NX" by Koukyu Alcohol Kogyo Co., Ltd.

Table 5

| | | Kind | Average Primary Particle Diameter $d_A$ (nm) | Average Particle Diameter $D_B$ (μm) | Disintegrability | Example 13 Formulation Example 1 | Example 14 Formulation Example 2 |
|---|---|---|---|---|---|---|---|
| Formulation of External Preparation (part) | Component (A) | Fine Particles of octyltriethoxysilane-treated titanium oxide A3 | 10 | - | - | 7 | 4 |
| | Component (B) | Composite Particles B1-7 | - | 3 | non-disintegrable | 7.5 | |
| | | Composite Particles B1-1 | - | 3 | non-disintegrable | | 1.5 |
| | | Porous Particles B2-2 | - | 5 | non-disintegrable | | 1 |
| | Oily Agent | Isopropyl Palmitate | | | | 4 | 4 |
| | | 2-Ethylhexyl Paramethoxycinnamate *1 | | | | 10 | 10 |
| | | 2,4-Bis[{4-(2-ethylhexyloxy)-2-hydroxy}-phenyl]-6-(4-methoxyphenyl)-1,3,5-triazine*2 | | | | 1 | 1 |
| | | 2,4,6-Tris[4-(2-ethylhexyloxycarbonyl)anilino]-1,3,5-triazine*3 | | | | 2 | 2 |
| | | Liquid Isoparaffin*4 | | | | 2 | 1 |
| | | Dimethylpolysiloxane*5 | | | | 1 | 2 |
| | | Cetanol*6 | | | | 0.5 | 0.5 |
| | Thickener/ Emulsifier | Acrylic Acid/Alkyl Methacrylate Copolymer 1 | | | | 0.25 | 0.25 |
| | | Acrylic Acid/Alkyl Methacrylate Copolymer 2 | | | | 0.2 | 0.2 |
| | Neutralizer/ Other Components | Potassium Hydroxide | | | | 0.6 | 0.6 |
| | | 1,3-Butylene Glycol | | | | | 3.5 |
| | | Phenoxyethanol | | | | 0.5 | 0.5 |
| | | EDTA-2 Na | | | | 0.01 | 0.01 |
| | Solvent | Ethanol | | | | 5 | 5 |
| | | Pure Water | | | | 58.44 | 62.94 |
| | Total | | | | | 100 | 100 |
| Mass Ratio[(A)/(B)] | | | | | | 0.93 | 1.60 |
| Evaluation Results | | Adhesion Suppressing Effect | Coating Amount of Component (A) (mg/cm²) | | | 0.14 | 0.08 |
| | | | Adhesion Suppressing Effect (%) | | | 86 | 80 |
| | | Feel (dry silky feel) | | | | 4.0 | 3.7 |

From Table 5, it is known that, in Examples 13 and 14, the external preparation containing a metal oxide having an average primary particle diameter $d_A$ falling within a predetermined range as the component (A) and non-disintegrable particles having an average particle diameter $D_B$ falling within a predetermined range as the component (B) in a predetermined mass ratio was applied, and therefore, the samples had a high adhesion suppressing effect and gave a good feel (dry silky feel) after application to skin.

Industrial Applicability

The adhesion suppressing method of the present invention provides a high adhesion suppressing effect against air harmful substances, and is therefore especially useful as a method of suppressing adhesion of air harmful substances to skin.

Reference Signs List

1: Sample Targeted for Evaluation
2: Support
3: Wire Sieve
4: Air Harmful Substances for Evaluation
5: Fan

## Claims

**1.** A method for suppressing adhesion of air harmful substances, comprising applying an external preparation to skin to suppress adhesion of air harmful substances to the skin, wherein:
the external preparation comprises the following component (A) and component (B), the content of the component (A) in the external preparation is 2 to 10% by mass, and the ratio by mass of the content of the component (A) to the content of the component (B) in the external preparation [(A)/(B)] is 0.30 or more and 5.0 or less:

Component (A): a metal oxide having an average primary particle diameter $d_A$ of 10 to 50 nm,
Component (B): non-disintegrable particles having an average particle diameter $D_B$ of 1 μm or more and 10 μm or less,
the component (A) is surface-hydrophobized,

the component (B) is at least one selected from the group consisting of

composite particles (B1) formed by coating at least a part of the surfaces of core particles (b1-1) that are at least one selected from the group consisting of poly(meth)acrylate particles and silica particles with inorganic fine particles (b1-2) that are at least one selected from the group consisting of titanium oxide and zinc oxide surface-treated with at least one selected from the group consisting of silica, hydrous silica and aluminum hydroxide, and
porous particles (B2) formed by aggregation of inorganic fine particles (b2) having an average primary particle diameter of 1 nm or more and 30 nm or less.

**2.** The method for suppressing adhesion of air harmful substances according to claim 1, wherein the coating amount of the component (A) on skin is 0.03 $mg/cm^2$ or more.

**3.** The method for suppressing adhesion of air harmful substances according to claim 1 or 2, wherein the component (A) is at least one selected from the group consisting of titanium oxide and zinc oxide.

**4.** The method for suppressing adhesion of air harmful substances according to any one of claims 1 to 3, wherein the coating amount of the component (A) on skin is 0.8 $mg/cm^2$ or less.

**5.** The method for suppressing adhesion of air harmful substances according to any one of claims 1 to 4, wherein the surfaces of the core particles (b1-1) constituting the composite particles (B1) are coated with at least one kind of binder (c) selected from the group consisting of a poly(N-vinylpyrrolidone), a poly(meth)acrylamide, and a homopolymer or a copolymer of oxazolines.

**6.** The method for suppressing adhesion of air harmful substances according to any one of claims 1 to 5, wherein the inorganic fine particles (b2) are particles comprising at least one selected from the group consisting of silica and titanium oxide.

**7.** An external preparation comprising the following component (A) and component (B):

Component (A): a metal oxide having an average primary particle diameter $d_A$ of 10 to 50 nm,
Component (B): non-disintegrable particles having an average particle diameter $D_B$ of 1 $\mu$m or more and 10 $\mu$m or less, wherein:

the component (A) is surface-hydrophobized,
the component (B) is composite particles (B1) formed by coating at least a part of the surfaces of core particles (b1-1) with inorganic fine particles (b1-2),
the core particles (b1-1) are at least one selected from the group consisting of poly(meth)acrylate particles and silica particles,
the surfaces of the core particles (b1-1) are coated with at least one kind of binder (c) selected from the group consisting of a poly(N-vinylpyrrolidone), a poly(meth)acrylamide, and a homopolymer or a copolymer of oxazolines,
the inorganic fine particles (b1-2) are at least one selected from titanium oxide and zinc oxide as surface-treated with one or more selected from the group consisting of silica, hydrous silica and aluminum hydroxide,
the content of the component (A) in the external preparation is 2 to 10% by mass, and
the ratio by mass of the content of the component (A) to the content of the component (B), [(A)/(B)] is 0.30 or more and 5.0 or less.

## Patentansprüche

**1.** Verfahren zur Unterdrückung der Adhäsion von Luftschadstoffen, umfassend das Auftragen einer externen Zubereitung auf die Haut, um die Adhäsion von Luftschadstoffen an der Haut zu unterdrücken, wobei:

die externe Zubereitung die folgende Komponente (A) und die folgende Komponente (B) umfasst, der Gehalt der Komponente (A) in der externen Zubereitung 2 bis 10 Massen-% beträgt und das Massenverhältnis von dem Gehalt der Komponente (A) zu dem Gehalt der Komponente (B) in der externen Zubereitung [(A)/(B)] 0,30 oder mehr und 5,0 oder weniger beträgt:

Komponente (A): ein Metalloxid mit einem durchschnittlichen Primärpartikeldurchmesser $d_A$ von 10 bis 50 nm,
Komponente (B): nicht desintegrierende Partikel mit einem durchschnittlichen Partikeldurchmesser $D_B$ von 1 $\mu$m oder mehr und 10 $\mu$m oder weniger,
die Komponente (A) oberflächenhydrophobisiert ist,
die Komponente (B) mindestens eines ist, ausgewählt aus der Gruppe, bestehend aus

Verbundpartikeln (B1), gebildet durch Beschichten mindestens eines Teils der Oberflächen von Kernpartikeln (b1-1), die mindestens eines, ausgewählt aus der Gruppe, bestehend aus Poly(meth)acrylat-Partikeln und Siliciumoxid-Partikeln, sind, mit anorganischen Feinpartikeln (b1-2), die mindestens eines, ausgewählt aus der Gruppe, bestehend aus Titanoxid und Zinkoxid, oberflächenbehandelt mit mindestens einem, ausgewählt aus der Gruppe, bestehend aus Siliciumoxid, wasserhaltigem Siliciumoxid und Aluminiumhydroxid, sind, und
porösen Partikeln (B2), gebildet durch Aggregieren von anorganischen Feinpartikeln (b2) mit einem durchschnittlichen Primärpartikeldurchmesser von 1 nm oder mehr und 30 nm oder weniger.

**2.** Verfahren zur Unterdrückung der Adhäsion von Luftschadstoffen gemäß Anspruch 1, wobei die Beschichtungsmenge der Komponente (A) auf der Haut 0,03 mg/cm$^2$ oder mehr beträgt.

**3.** Verfahren zur Unterdrückung der Adhäsion von Luftschadstoffen gemäß Anspruch 1 oder 2, wobei die Komponente (A) mindestens eine, ausgewählt aus der Gruppe, bestehend aus Titanoxid und Zinkoxid, ist.

**4.** Verfahren zur Unterdrückung der Adhäsion von Luftschadstoffen gemäß einem der Ansprüche 1 bis 3, wobei die Beschichtungsmenge der Komponente (A) auf der Haut 0,8 mg/cm$^2$ oder weniger beträgt.

**5.** Verfahren zur Unterdrückung der Adhäsion von Luftschadstoffen gemäß einem der Ansprüche 1 bis 4, wobei die Oberflächen der Kernpartikel (b1-1), die die Verbundpartikel (B1) bilden, mit mindestens einer Art von Bindemittel (c) beschichtet sind, das aus der Gruppe ausgewählt ist, die aus einem Poly(N-vinylpyrrolidon), einem Poly(meth)acrylamid und einem Homopolymer oder einem Copolymer von Oxazolinen besteht.

**6.** Verfahren zur Unterdrückung der Adhäsion von Luftschadstoffen gemäß einem der Ansprüche 1 bis 5, wobei die anorganischen Feinpartikel (b2) Partikel sind, die mindestens eines, ausgewählt aus der Gruppe, bestehend aus Siliciumoxid und Titanoxid, umfassen.

**7.** Externe Zubereitung, umfassend die folgenden Komponenten (A) und (B):

Komponente (A): ein Metalloxid mit einem durchschnittlichen Primärpartikeldurchmesser $d_A$ von 10 bis 50 nm,
Komponente (B): nicht desintegrierende Partikel mit einem durchschnittlichen Partikeldurchmesser $D_B$ von 1 $\mu$m oder mehr und 10 $\mu$m oder weniger, wobei:

die Komponente (A) oberflächenhydrophobisiert ist,
die Komponente (B) Verbundpartikel (B1), gebildet durch Beschichten mindestens eines Teils der Oberflächen von Kernpartikeln (b1-1) mit anorganischen Feinpartikeln (b1-2), ist,
die Kernpartikel (b1-1) mindestens eines, ausgewählt aus der Gruppe, bestehend aus Poly(meth)acrylat-Partikeln und Siliciumoxid-Partikeln, sind,
die Oberflächen der Kernpartikel (b1-1) mit mindestens einer Art von Bindemittel (c) beschichtet sind, das aus der Gruppe ausgewählt ist, die aus einem Poly(N-vinylpyrrolidon), einem Poly(meth)acrylamid und einem Homopolymer oder einem Copolymer von Oxazolinen besteht,
die anorganischen Feinpartikel (b1-2) mindestens eines, ausgewählt aus Titanoxid und Zinkoxid, oberflächenbehandelt mit einem oder mehreren, ausgewählt aus der Gruppe, bestehend aus Siliciumoxid, wasserhaltigem Siliciumoxid und Aluminiumhydroxid, sind, der Gehalt der Komponente (A) in der externen Zubereitung 2 bis 10 Massen-% beträgt, und
das Massenverhältnis von dem Gehalt der Komponente (A) zu dem Gehalt der Komponente (B), [(A)/(B)], 0,30 oder mehr und 5,0 oder weniger beträgt.

## Revendications

**1.** Procédé pour supprimer l’adhérence de substances nocives présentes dans l’air, comprenant une application d’une

préparation externe sur la peau afin de supprimer l'adhérence de substances nocives présentes dans l'air sur la peau, dans lequel :
la préparation externe comprend les composants (A) et (B) suivants, la teneur en composant (A) dans la préparation externe est comprise entre 2 et 10 % en masse, et le rapport massique entre la teneur en composant (A) et la teneur en composant (B) dans la préparation externe [(A)/(B)] est compris entre 0,30 et 5,0 :

Composant (A) : un oxyde métallique présentant un diamètre moyen de particules primaires $d_A$ compris entre 10 et 50 nm,
Composant (B) : des particules non désintégrables présentant un diamètre moyen de particules $D_B$ compris entre 1 $\mu$m et 10 $\mu$m,
le composant (A) est hydrophobisé en surface,
le composant (B) est au moins un composant sélectionné parmi le groupe constitué par

des particules composites (B1)formées en revêtant au moins une partie des surfaces de particules centrales (b1-1) qui sont au moins un type de particules sélectionné parmi le groupe constitué par les particules de poly(méth)acrylate et les particules de silice avec des particules fines inorganiques (b1-2) qui sont au moins un type de particules sélectionné parmi le groupe constitué par l'oxyde de titane et l'oxyde de zinc, qui sont traitées en surface avec au moins un élément sélectionné parmi le groupe constitué par la silice, la silice hydratée et l'hydroxyde d'aluminium, et
des particules poreuses (B2) formées par agrégation de particules fines inorganiques (b2) présentant un diamètre moyen de particules primaires compris entre 1 nm et 30 nm.

**2.** Procédé pour supprimer l'adhérence de substances nocives présentes dans l'air selon la revendication 1, dans lequel la quantité de revêtement du composant (A) sur la peau est supérieure ou égale à 0,03 mg/cm$^2$.

**3.** Procédé pour supprimer l'adhérence de substances nocives présentes dans l'air selon la revendication 1 ou la revendication 2, dans lequel le composant (A) est au moins un composant sélectionné parmi le groupe constitué par l'oxyde de titane et l'oxyde de zinc.

**4.** Procédé pour supprimer l'adhérence de substances nocives présentes dans l'air selon l'une quelconque des revendications 1 à 3, dans lequel la quantité de revêtement du composant (A) sur la peau est inférieure ou égale à 0,8 mg/cm$^2$.

**5.** Procédé pour supprimer l'adhérence de substances nocives présentes dans l'air selon l'une quelconque des revendications 1 à 4, dans lequel les surfaces des particules centrales (b1-1) constituant les particules composites (B1) sont revêtues d'au moins un type de liant (c) sélectionné parmi le groupe constitué par une poly(N-vinylpyrrolidone), un poly(méth)acrylamide et un homopolymère ou un copolymère d'oxazolines.

**6.** Procédé pour supprimer l'adhérence de substances nocives présentes dans l'air selon l'une quelconque des revendications 1 à 5, dans lequel les particules fines inorganiques (b2) sont des particules comprenant au moins un type de particules sélectionné parmi le groupe constitué par la silice et l'oxyde de titane.

**7.** Préparation externe comprenant les composants (A) et (B) suivants :

Composant (A) : un oxyde métallique présentant un diamètre moyen de particules primaires $d_A$ compris entre 10 et 50 nm,
Composant (B) : des particules non désintégrables présentant un diamètre moyen de particules $D_B$ compris entre 1 $\mu$m et 10 $\mu$m, dans laquelle :

le composant (A) est hydrophobisé en surface,
le composant (B) est constitué par des particules composites (B1) formées en revêtant au moins une partie des surfaces de particules centrales (b1-1) avec des particules fines inorganiques (b1-2),
les particules centrales (b1-1) sont au moins un type de particules sélectionné parmi le groupe constitué par les particules de poly(méth)acrylate et les particules de silice,
les surfaces des particules centrales (b1-1) sont revêtues d'au moins un type de liant (c) sélectionné parmi le groupe constitué d'une poly(N-vinylpyrrolidone), d'un poly(méth)acrylamide et d'un homopolymère ou d'un copolymère d'oxazolines,
les particules fines inorganiques (b1-2) au moins un type de particules sélectionné parmi le groupe constitué

par l'oxyde de titane et l'oxyde de zinc, qui sont traitées en surface avec au moins un élément sélectionné parmi le groupe constitué par la silice, la silice hydratée et l'hydroxyde d'aluminium,
la teneur en composant (A) dans la préparation externe est comprise entre 2 et 10 % en masse, et
le rapport massique entre la teneur en composant (A) et la teneur en composant (B), [(A)/(B)] est compris entre 0,30 et 5,0.

Fig. 1

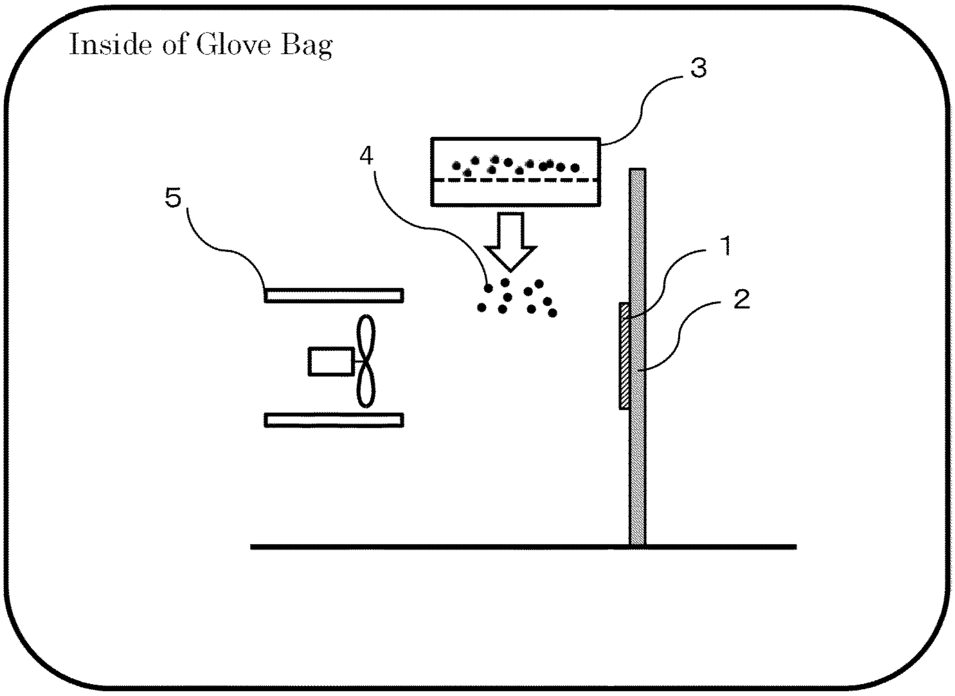

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014136993 A
- JP 2005336056 A
- JP 2006008980 A
- JP 2005162700 A
- JP 2016006029 A

### Non-patent literature cited in the description

- *Nature Chemistry*, 2011, vol. 3, 291-295